(19) **Europäisches Patentamt** / **European Patent Office** / **Office européen des brevets**

(11) **EP 2 552 844 B1**

(12) **FASCICULE DE BREVET EUROPEEN**

(45) Date de publication et mention de la délivrance du brevet:
**26.03.2014 Bulletin 2014/13**

(51) Int Cl.:
**G01N 21/35** *(2014.01)*  **G01N 21/55** *(2014.01)*
**C03C 13/04** *(2006.01)*  **C03B 37/15** *(2006.01)*

(21) Numéro de dépôt: **11711367.0**

(22) Date de dépôt: **31.03.2011**

(86) Numéro de dépôt international:
**PCT/EP2011/055038**

(87) Numéro de publication internationale:
**WO 2011/121086 (06.10.2011 Gazette 2011/40)**

(54) **PROCÉDÉ DE FABRICATION D'UN CAPTEUR À ONDES INFRAROUGES ÉVANESCENTES À FIBRE DE CHALCOGÉNURE**

VERFAHREN ZUR HERSTELLUNG EINES CHALKOGENIDFASER-INFRAROT-EVANESZENZWELLENSENSORS

METHOD OF MANUFACTURING A CHALCOGENIDE-FIBRE INFRARED EVANESCENT WAVE SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.04.2010 FR 1052457**

(43) Date de publication de la demande:
**06.02.2013 Bulletin 2013/06**

(73) Titulaires:
- **Université de Rennes 1**
  **35000 Rennes (FR)**
- **Centre National de la Recherche Scientifique (CNRS)**
  **75016 Paris (FR)**

(72) Inventeurs:
- **BUREAU, Bruno**
  **F-35510 Cesson (FR)**
- **BOUSSARD, Catherine**
  **F-35830 Betton (FR)**
- **LOREAL, Olivier**
  **F-35170 Bruz (FR)**
- **HOUIZOT, Patrick**
  **F-35580 Guignen (FR)**
- **ADAM, Jean-Luc**
  **F-35000 Rennes (FR)**
- **LUCAS, Jacques**
  **F-35830 Betton (FR)**

(74) Mandataire: **Regimbeau**
  **20, rue de Chazelles**
  **75847 Paris Cedex 17 (FR)**

(56) Documents cités:
**WO-A1-2006/045443    JP-A- 7 237 932**
**US-A- 5 525 800**

- HOCDE S ET AL: "Recent developments in chemical sensing using infrared glass fibers", JOURNAL OF NON-CRYSTALLINE SOLIDS, NORTH-HOLLAND PHYSICS PUBLISHING. AMSTERDAM, NL LNKD- DOI: 10.1016/S0022-3093(00)00179-4, vol. 274, no. 1-3, 1 septembre 2000 (2000-09-01), pages 17-22, XP004214791, ISSN: 0022-3093
- KEIRSSE J ET AL: "IR optical fiber sensor for biomedical applications", VIBRATIONAL SPECTROSCOPY, ELSEVIER SCIENCE, AMSTERDAM, NL, vol. 32, no. 1 special, 5 août 2003 (2003-08-05), pages 23-32, XP007915702, ISSN: 0924-2031, DOI: DOI:10.1016/S0924-2031(03)00044-4 [extrait le 2003-05-23]
- LITTLEJOHN D ET AL: "BENT SILICA FIBER EVANESCENT ABSORPTION SENSORS FOR NEAR-INFRARED SPECTROSCOPY", APPLIED SPECTROSCOPY, THE SOCIETY FOR APPLIED SPECTROSCOPY. BALTIMORE, US, vol. 53, no. 7, 1 juillet 1999 (1999-07-01), pages 845-849, XP000835472, ISSN: 0003-7028, DOI: DOI: 10.1366/0003702991947423

- **WIKIPEDIA: "Glass Transition", INTERNET CITATION, 10 novembre 2010 (2010-11-10), pages 1-10, XP007915721, [extrait le 2010-11-10]**
- **HEISE H M ET AL: "Attenuated total reflection mid-infrared spectroscopy for clinical chemistry applications using silver halide fibers", SENSORS AND ACTUATORS B, ELSEVIER SEQUOIA S.A., LAUSANNE, CH, vol. 51, no. 1-3, 31 août 1998 (1998-08-31), pages 84-91, XP004153993, ISSN: 0925-4005, DOI: DOI: 10.1016/S0925-4005(98)00231-7**

**EP 2 552 844 B1**

**Description**

L'invention concerne un procédé de fabrication d'un capteur à ondes infrarouges évanescentes à fibre de chalcogénure.

Les verres de chalcogénure sont des verres à basse température de fusion, et basse température de transition vitreuse, et sont des matériaux très originaux, délicats à synthétiser et maîtriser. Leurs performances étendent fortement la sensibilité des capteurs FEWS (FEWS étant en anglais « Fiber Evanescent Wave Spectroscopy » soit spectroscopie à ondes évanescentes d'une fibre). Surtout, ces verres à base de Soufre, Sélénium et/ou tellure laissent passer la lumière sur une large gamme de longueur d'onde dans l'infrarouge ce qui n'est pas le cas des verres d'oxydes classiques. De plus, la nature vitreuse du matériau permet de le mettre en forme pour fabriquer des fibres optiques. Enfin, ce sont des verres faisant intervenir des éléments chimiques proches les uns des autres dans le tableau périodique entretenant donc entre eux des liaisons très covalentes. Cette nature des liaisons chimiques rend le matériau hydrophobe ce qui est très positif lors de leur utilisation comme capteur dans des milieux riches en eau tels les échantillons biologiques.

En effet, l'un des domaines d'application de ce capteur est biomédical.

Le document 'IR optical fiber sensor for biomédical applications" de J.Keirsse, C. Boussard-Plédel, O. Loréal, O. Sire, B. Bureau, P. Leroyer, B. Turlin, J. Lucas, Vibrational Spectroscopy 32 (2003), pages 23 à 32, décrit le principe d'un tel capteur dans le domaine de l'infrarouge moyen. Dans ce dispositif, une unique fibre de $Te_2As_3Se_5$ est utilisée comme guide d'ondes et comme élément de détection. Cette fibre a une fenêtre spectrale allant de 800 à 4000 cm$^{-1}$. Pour améliorer la sensibilité de ce capteur, son diamètre est localement réduit pour créer une zone de détection effilée en U qui est amenée en contact avec un échantillon devant être analysé. Le diamètre de cette fibre de 400 micromètres passe à 100 micromètres dans cette zone de détection. La fibre est couplée à un spectromètre FTIR. Le principe de mesure est d'envoyer dans la fibre une onde infrarouge qui se propagera le long de la fibre par réflexions successives sur sa surface extérieure. Dans la zone de détection en contact avec l'échantillon, l'onde évanescente se propageant le long de la paroi extérieure de la fibre est partiellement absorbée par la substance cible. Le spectromètre analyse l'onde reçue à l'autre extrémité de la fibre pour en déduire des informations sur l'échantillon.

Le document "Advances in chalcogenide fiber evanescent wave biochemical sensing" de Pierre Lucas, Mark R. Riley, Catherine Boussard-Plédel, Bruno Bureau, Analytical Biochemistry (2006), pages 1 à 10, décrit un capteur à fibre de chalcogénure, en précisant que le rayon de courbure de la fibre dépend de son diamètre et peut atteindre 2 centimètres pour une fibre de 100 micromètres.

Le document US 5525800 décrit une réalisation d'un tel capteur à base d'une fibre de chalcogénure, dédié à la détection d'une longueur d'onde comprise entre 2 et 12 $\mu$m. Ce document propose d'utiliser une fibre ayant un diamètre compris entre 20 et 500 $\mu$m et dont le coeur a un diamètre de l'ordre de 30 à 70% celui de la fibre. Plus précisément pour améliorer les propriétés du capteur, ce document propose que le coeur de la fibre ait un indice de réfraction supérieur à celui de la gaine, mais que la différence entre ces indices soit minimisée afin d'optimiser la part de rayonnement qui chemine dans la gaine et par conséquent la propagation d'ondes évanescentes. Une partie intermédiaire de la fibre dépourvue de gaine, est revêtue d'un matériau polymère ayant un indice inférieur à l'indice du coeur et de la gaine et présentant une affinité avec les espèces chimiques à détecter. Par ailleurs, ce document indique que le rayon de courbure minimal d'une fibre de chalcogénure d'un diamètre de 200 $\mu$m est de l'ordre de 1cm, sans préciser le processus de réalisation d'une telle courbure. Plus précisément encore ce document préconise, pour abaisser le seuil de détection d'un tel capteur, d'allonger la longueur de la région revêtue d'un polymère, d'utiliser un polymère présentant un facteur d'affinité enrichi, de réduire la section transversale du coeur et de réduire la différence entre les indices du coeur et du polymère.

Le document LITTLEJOHN D ET AL. : « BENT SILICA FIBER EVANESCENT ABSORPTION SENSORS FOR NEAR-INFRARED SPECTROSCOPY », vol. 53, n° 7, 1 juillet 1999, pages 845-849, XP000835472, décrit un capteur à base de fibre de silice.

Le document HOCDE S ET AL. : « Recent developments in chemical sensing using infrared glass fibers », JOURNAL OF NON-CRYSTALLINE SOLIDS, NORTH-HOLLAND PHYSICS PUBLISHING. AMSTERDAM, NL LNKD-DO1:10.1016/S0022-3093(00)00179-4, vol. 274, n°1-3, 1 septembre 2000, pages 17-22, XP004214791, est un article général portant sur un capteur à base de chalcogénure, qui est assez général et dont l'enseignement est proche de celui des deux premiers documents cités.

Le document JP 7237932 divulgue un procédé de courbure d'une fibre optique qui consiste à introduire la fibre dans un four et à courber la fibre à l'intérieur du four en l'enroulant autour d'un mandrin également placé dans le four.

Les problèmes posés par le capteur sont son manque de compacité, sa fragilité, et sa faible sensibilité dans sa zone de détection.

L'invention vise à obtenir un capteur utilisant au moins une fibre en verre de chalcogénure fonctionnant sur le principe de l'absorption par ondes évanescentes qui soit compact, robuste et sensible aux signatures dans le moyen infrarouge des molécules cibles.

L'objet de l'invention est un procédé de fabrication d'un capteur tel que défini en revendication 1 annexée.

Suivant un mode de réalisation de l'invention, l'âme comporte des première et deuxième parties, entre lesquelles se trouve ladite zone de contact, l'âme étant chauffée par au moins l'une des première et deuxième parties.

Suivant un mode de réalisation de l'invention, l'âme est cylindrique de section quelconque.

Suivant un mode de réalisation de l'invention, la température T2 dépasse la température de transition vitreuse Tg de 10 % à 20 %.

Suivant un mode de réalisation de l'invention, la partie intermédiaire a un diamètre (D2) de fibre compris entre 50 et 450 micromètres.

L'invention sera mieux comprise à la lecture de la description qui va suivre, donnée uniquement à titre d'exemple non limitatif en référence aux dessins annexés, sur lesquels :

- la figure 1 représente schématiquement en perspective en vue agrandie un premier mode de réalisation d'un capteur suivant l'invention,
- la figure 2 est une vue schématique en coupe longitudinale de la zone de détection du capteur suivant l'invention avant courbure, la partie intermédiaire ayant un diamètre pouvant être égal à celui des parties 1 et 3.
- les figures 3, 4 et 5 sont des vues schématiques en perspective agrandie de deuxième, troisième et quatrième modes de réalisation du capteur suivant l'invention,
- la figure 6 représente schématiquement un dispositif utilisant le capteur suivant l'invention,
- la figure 7 est une vue schématique en perspective agrandie d'un tube endoscopique utilisant le capteur suivant l'invention,
- la figure 8 est une vue schématique agrandie de côté d'une fibre du capteur suivant l'invention au cours d'une étape de son procédé de fabrication,
- la figure 9 est une vue schématique agrandie de dessus d'une fibre du capteur suivant l'invention au cours de l'étape de son procédé de fabrication selon la figure 8,
- la figure 10 est un diagramme de l'absorbance d'un exemple de fibre suivant l'invention en fonction de la longueur d'onde,
- la figure 11 est une vue schématique de la mesure de résistance mécanique de la partie courbe de la fibre de chalcogénure suivant l'invention,
- la figure 12 est une vue schématique de la mesure de résistance mécanique d'une fibre non mise en forme selon le procédé suivant l'invention.

Aux figures, le capteur 1 comporte une fibre 2 en verre de chalcogénure. La composition de la fibre est du type XY, où X est Ge, As ou Sb ou un mélange de deux ou de plus de deux de ces composants, et où Y est du S, Se, Te ou un mélange de deux ou plus de deux de ces composants. Par exemple, la proportion en poids de X est supérieure ou égale à 10% et inférieure ou égale à 70 %, tandis que la proportion en poids de Y est supérieure ou égale à 30 % et inférieure ou égale à 90 %. La fibre sert au guidage d'ondes infrarouges.

Dans un exemple de réalisation, la fibre est en $As_2Se3$.

Dans un autre exemple de réalisation, la fibre est en $Te_2As_3Se_5$.

La fibre 2 du capteur 1 s'étend suivant une direction générale longitudinale L entre une première extrémité 21 et une deuxième extrémité 22. La fibre 2 comporte, de la première extrémité 21 à la deuxième extrémité 22, un premier tronçon 23 de fibre pour le guidage d'ondes infrarouges, ce premier tronçon 23 étant raccordé de son côté éloigné de la première extrémité 21 à un premier point 24 d'un deuxième tronçon 25 de détection, lequel deuxième tronçon 25 est raccordé par un deuxième point 26 éloigné du premier point 24 à un troisième tronçon 27 de fibre pour le guidage des ondes infrarouges.

Dans un mode de réalisation, le deuxième tronçon 25 de détection de la fibre 2 présente une largeur transversale D1 dans au moins une dimension, un diamètre D1 ou une section transversale D1, qui sont inférieurs à la largeur transversale D2, ou au diamètre D2 ou à la section transversale D2 du premier tronçon 23, et qui sont inférieurs à la largeur transversale D2, au diamètre D2 ou à la section transversale D2 du troisième tronçon 27. Le deuxième tronçon 25 de détection de la fibre 2 s'étend sur une longueur L1 non nulle de la fibre entre les premiers et deuxièmes points 24, 26 de raccordement et entre le premier tronçon 23 et le troisième tronçon 27.

Par exemple, la fibre 2 a un diamètre moyen D2 de 400 micromètres environ dans le premier tronçon 23 et dans le troisième tronçon 27, tandis que la fibre 2 a dans le deuxième tronçon 25 de détection un diamètre moyen D1 de 100 micromètres environ pour une longueur L1 du deuxième tronçon de détection de 10 centimètres environ. Le premier tronçon 23 a une longueur supérieure à la longueur du deuxième tronçon 25 de détection. Le troisième tronçon 27 a une longueur supérieure à la longueur L1 du deuxième tronçon 25 de détection.

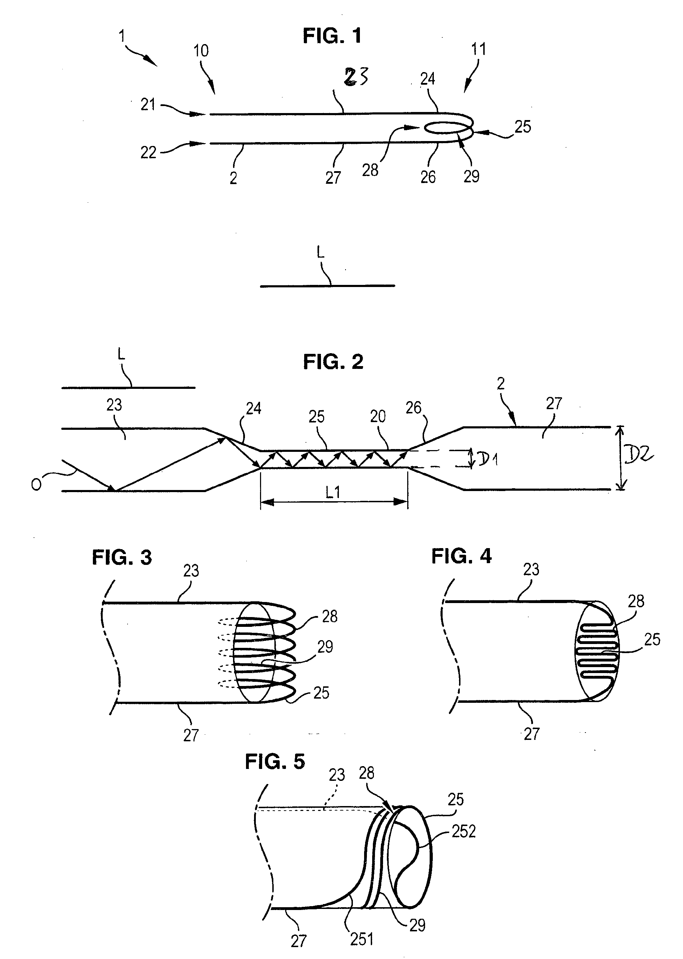

Le premier point 24 de raccordement est par exemple formé par une zone de section transversale s'effilant du premier tronçon 23 au deuxième tronçon 25 de détection, en étant par exemple tronconique ainsi que représenté à la figure 2. Le deuxième point 26 de raccordement a par exemple une section transversale s'élargissant du deuxième tronçon 25 de détection au troisième tronçon 27, en étant par exemple de forme tronconique ainsi que représenté à la figure 2. Entre les premier et deuxième points 24, 26 de raccordement, le deuxième tronçon 25 de détection a par exemple une largeur transversale D1 constante et/ou un diamètre transversal D1 constant et/ou une section transversale D1 constante.

Dans un autre mode de réalisation, le deuxième tronçon 25 de détection de la fibre 2 présente une largeur transversale (ou épaisseur) dans au moins une dimension, un diamètre ou une section transversale, qui sont égaux à la largeur transversale, ou au diamètre ou à la section transversale du premier tronçon et/ou qui sont égaux à la largeur transversale (ou épaisseur), au diamètre ou à la section transversale du troisième tronçon 27. La fibre 2 a par exemple une section transversale et/ou un diamètre constant dans les premier, deuxième et troisième tronçons 23, 25 et 27.

Dans un mode de réalisation, la fibre a par exemple sur les premier, deuxième et troisième tronçons 23, 25 et 27 un diamètre (ou épaisseur) de fibre compris entre 50 et 450 micromètres.

Par exemple, dans un mode de réalisation, la fibre a sur les premier, deuxième et troisième tronçons 23, 25 et 27 un diamètre (ou épaisseur) de fibre compris entre 50 et 450 micromètres et constant sur ces premier, deuxième et troisième tronçons 23, 25 et 27.

Le deuxième tronçon 25 de détection est destiné à venir en contact avec un milieu extérieur pour détecter les perturbations apportées par ce milieu extérieur à la propagation des ondes infrarouges dans la fibre 2 et forme une tête de détection du capteur. Les premier et deuxième tronçons 23, 27 sont des tronçons d'amenée des ondes infrarouge.

La figure 2 montre, par des flèches épaisses à l'intérieur de la fibre 2 de manière symbolique, le trajet d'une onde infrarouge O envoyée par la première extrémité 21 dans le tronçon 23. Du fait du rétrécissement de la section transversale de la fibre 2 dans le deuxième tronçon 25 de détection, il y a plus de réflexions de l'onde dans ce deuxième tronçon 25 contre la surface extérieure 20 de la fibre 2.

Lorsqu'un milieu extérieur est mis en contact avec le deuxième tronçon 25 de détection, la propagation de l'onde O dans ce deuxième tronçon 25 est perturbée, du fait qu'une partie de l'onde O passe du deuxième tronçon 25 vers le milieu extérieur.

Par conséquent, le contact du deuxième tronçon 25 de détection avec un milieu extérieur aura une influence sur l'onde O qui sera transmise depuis le premier tronçon 23 au troisième tronçon 27.

Suivant l'invention, le deuxième tronçon 25 de détection comporte au moins une partie courbe 28 de fibre ayant un rayon de courbure de fibre inférieur à 2,3 millimètres.

La partie courbe 28 est par exemple sous la forme d'un enroulement ayant une ou plusieurs spires, ou a par exemple une forme solénoïdale. Dans un autre exemple, la partie courbe 28 peut avoir une forme en U. Le deuxième tronçon est par exemple en forme de boucle ouverte. On peut bien entendu avoir un nombre quelconque de partie courbes 28, par exemple n boucles ouvertes de géométries quelconques, $n \geq 1$.

A la figure 2, le deuxième tronçon 25 de détection est représenté de manière rectiligne avant d'être courbé pour former la partie courbe 28.

Par exemple, après courbure, les tronçons 23 et 27 se font face. Les tronçons 23 et 27 sont par exemple parallèles entre eux.

Par exemple, le rayon de courbure de la partie courbe 28 du deuxième tronçon 25 est inférieur ou égal à 1.4 mm et notamment inférieur ou égal à 1 mm.

Par exemple, dans les modes de réalisation décrits aux figures, le premier tronçon 23 de fibre et le troisième tronçon 25 de fibre sont distants l'un de l'autre d'une largeur inférieure à 2,8 mm transversalement à la longueur de la fibre et occupent un espace d'une largeur inférieure à 2,8 mm transversalement à la longueur de la fibre.

Des modes de réalisation du deuxième tronçon 25 sont représentés aux figures 1, 3, 4 et 5.

Dans les modes de réalisation des figures 1, 3 et 5, la partie courbe 28 est en forme d'enroulement ayant une ou plusieurs spires 29 par exemple circulaires.

Dans le mode de réalisation de la figure 1, la partie courbe 28 est formée d'une spire et demie 29 et se trouve à peu près dans le plan général des tronçons 23 et 27, l'enroulement 28 ayant été représenté dilaté en hauteur à la figure 1 pour montrer les différentes parties de l'enroulement 28. D'une manière générale, la ou les spires 29 de l'enroulement peuvent se trouver chacune dans un plan.

Dans le mode de réalisation de la figure 3, les spires 29 de l'enroulement de la partie 28 sont en forme générale d'hélice raccordée aux tronçons 23 et 27 se faisant face et par exemple parallèles entre eux.

Une seule spire hélicoïdale, ou une spire et demie hélicoïdale 29 pourrait également être prévue.

Dans le mode de réalisation de la figure 4, la partie courbe 28 forme des méandres en changeant de convexité au moins une fois, par exemple dans un plan.

Dans le mode de réalisation de la figure 5, la partie courbe 28 comporte un enroulement ayant une ou plusieurs spires 29 faisant le tour d'un cylindre géométrique le long duquel se trouvent les tronçons 23 et 27, le cylindre géométrique sur lequel se trouve l'enroulement étant par exemple circulaire. Les parties 251, 252 de raccordement des spires 29 aux tronçons 23 et 27 ont également le rayon de courbure inférieur à 2,3 millimètres, qui est par exemple égal à 0.8 mm pour un rayon de courbure de 1 mm environ des spires 29 autour du cylindre géométrique. Le diamètre du cylindre géométrique autour duquel est enroulé la fibre 2 est compris entre 1.5 et 4 mm.

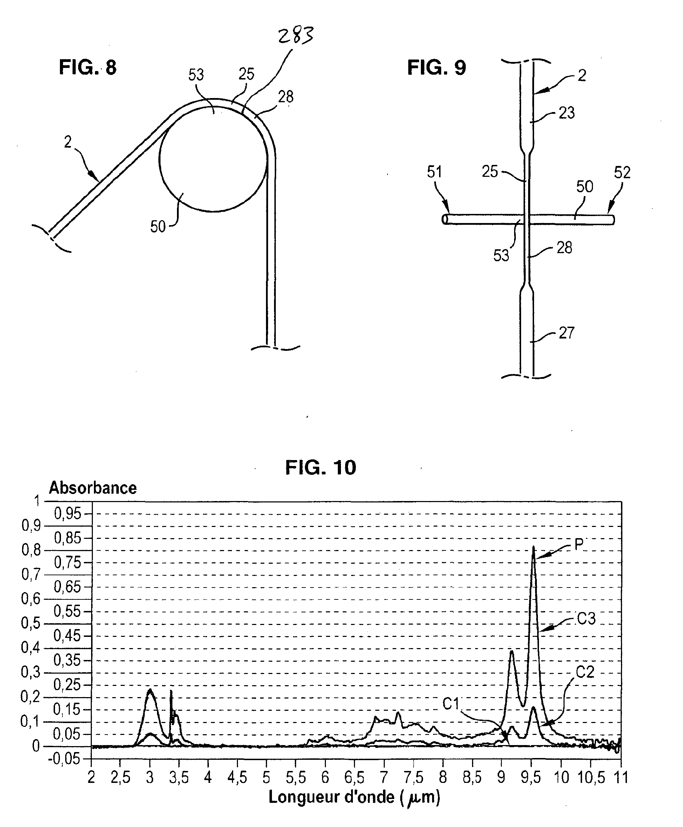

Un procédé permettant de réaliser la partie courbe 28 dans le deuxième tronçon 25 de détection de la figure 2 sera décrit ci-après en référence aux figures 8 et 9.

L'effilement 24, 26 est facultatif dans la réalisation de la tête 28. Pour fabriquer le capteur 1 suivant l'invention, on étire une fibre de verre de chalcogénure pour avoir un diamètre moyen déterminé de celle-ci, par exemple de 400 micromètres pour former les premier et troisième tronçons 23, 27. Le deuxième tronçon 25 de largeur transversale inférieure à celle des tronçons 23, 27 dans au moins une dimension est formé par exemple par une accélération de la vitesse d'étirage, une attaque chimique par une solution d'acide sulfurique et d'eau oxygénée ou encore par écrasement à chaud à une température égale à Tg + 15 %, $T_g$ étant la température de transition vitreuse de la matière de la fibre 2. On obtient par exemple un tronçon 25 pouvant être réduit à moins de 100 micromètres dans au moins une dimension transversale.

La température $T_g$ de transition vitreuse est mesurée par analyse calorimétrique différentielle, avec une montée en température de 10 °C par minute.

La fibre 2 s'étend alors suivant la direction longitudinal L comme à la figure 2.

Le tronçon 25 est ensuite courbé par formage à chaud. Pour ce faire, on applique le tronçon 25 de la fibre 2 contre une âme 50 chauffée pour que la partie intermédiaire 25 qui est mise en contact avec l'âme 50 soit à une température $T_2$ telle que :

$$1{,}05 \, . \, \mathrm{Tg} \leq \mathrm{T2} \leq 1{,}5 \, . \, \mathrm{Tg} \ ,$$

et notamment

$$1{,}1 \, . \, \mathrm{T_g} \leq \mathrm{T_2} \leq 1{,}2 \, . \, \mathrm{T_g}.$$

Dans un exemple de réalisation, on dépasse la température de transition vitreuse Tg de 10 % à 15 % pour chauffer le tronçon 25 devant être courbé.

La zone 53 de contact de l'âme 50 contre laquelle est appliquée le tronçon 25 a des dimensions transversales (par exemple diamètre) inférieures à 4.6 mm pour l'enroulement du tronçon 25 suivant le rayon de courbure imposé par cette zone 53 de contact. La zone 53 de contact de l'âme 50 a par exemple des dimensions transversales (par exemple diamètre) inférieures ou égales à 2.4 mm et par exemple inférieures ou égales à 2 mm. Les dimensions transversales ou le diamètre de la zone 53 autour de laquelle est enroulée le tronçon 25 de la fibre 2 est par exemple compris entre 1.5 et 4 mm.

D'une manière générale pour le verre de chalcogénure utilisé, la température de transition vitreuse $T_g$ est supérieure ou égale à 90°C et inférieure ou égale à 400°C.

Le chauffage de l'âme 50 a par exemple lieu en deux première et deuxième parties 51, 52 de celle-ci, entre lesquelles se trouve une troisième zone 53 de contact contre laquelle est appliquée le tronçon intermédiaire 25 de la fibre 2. Le chauffage de cette troisième zone 53 se fait par exemple par conduction thermique depuis les parties 51, 52, l'âme étant par exemple métallique, des moyens de chauffage de l'âme 50 étant prévus. L'âme 50 est par exemple de forme oblongue entre les parties 51, 52. Au moins dans sa zone 53 de contact ou entièrement, l'âme 50 est par exemple de forme cylindrique, notamment cylindrique circulaire. Bien entendu, l'âme 50 pourrait n'être chauffée qu'à partir d'une seule des deux parties 51,52.

Pour réaliser la courbure, on applique le tronçon intermédiaire 25 contre l'âme 50 ainsi chauffée et ayant le rayon de courbure souhaité. Cette âme est par exemple en acier inoxydable et a par exemple une section circulaire pour former un enroulement circulaire dans le tronçon 25. On enroule ainsi le tronçon 25 d'une manière contrôlée autour de l'âme 50 chauffée pour former la partie courbe, par exemple une ou plusieurs spires de l'enroulement 28, c'est-à-dire en faisant au moins 360° autour de l'âme 50, la partie courbe 28 étant donc courbée avec le rayon de courbure de l'âme 50.

Puis, on refroidit le tronçon 25 d'une manière contrôlée, par exemple de 2°C par minute et on retire l'âme 50.

On a ainsi fabriqué plusieurs parties courbes 28 formées d'un enroulement compris entre une et cinq spires plus une demie selon la figure 1, ayant un rayon de courbure de 1 mm dans le tronçon 25 ayant un diamètre D1 de fibre de 100 micromètres, les tronçons 23 et 27 ayant un diamètre D2 de fibre de 400 micromètres, la fibre étant de section transversale circulaire. Le modèle préféré sera un modèle à trois spires, au-delà, la longueur du solénoide (dimension parallèle à l'axe de symétrie) devient supérieure au diamètre des spires et affecte la compacitié de l'ensemble.

Ainsi, c'est l'âme 50 autour de laquelle la fibre va être enroulée, qui est chauffée, afin de transmettre sa chaleur à la fibre. On obtient ainsi un capteur à fibre de chalcogénure ayant une meilleure résistance mécanique dans la partie courbe 28, du fait que la surface intérieure 283, ayant été chauffée, de la partie courbe 28 a subi un recuit par ce chauffage localisé.

La mise en forme à chaud effectuée par ce chauffage localisé par l'âme 50 renforce la résistance mécanique du capteur à fibre de chalcogénure de façon significative en éliminant les tensions résiduelles générées lors de la flexion de la fibre. Ainsi, les inventeurs ont déterminé une résistance de 4 Newton pour une partie courbe 28 de trois spires ayant un diamètre de fibre de 200 $\mu$m et un rayon de courbure de 1 mm selon la figure 1 (partie courbe 28 formée d'un enroulement de trois spires et demie).

Dans un mode de réalisation, l'âme chauffée 50 autour de laquelle on enroule la fibre comporte un coeur en cuivre ou en acier Inox, recouvert d'une couche extérieure en Téflon, pour empêcher la fibre de coller sur l'âme 50. D'une manière générale, l'âme 50 en un premier matériau métallique est recouverte d'une couche extérieure en un deuxième matériau différent et anti-adhésif pour la fibre, tel que par exemple en Téflon.

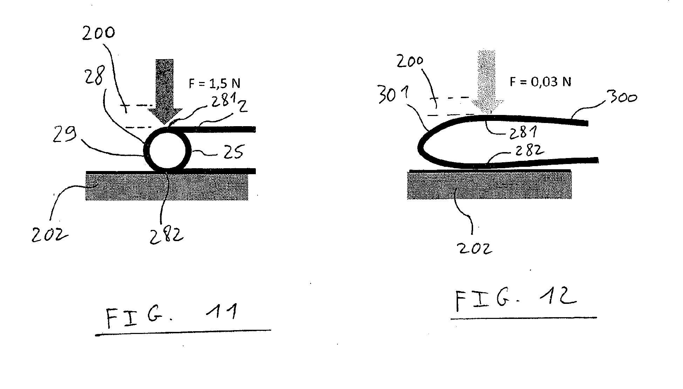

La manière dont a été réalisée cette mesure de résistance mécanique de la partie courbe 28 de la fibre de chalcogénure suivant l'invention est la même que celle représentée à la figure 11 et est décrite ci-dessous.

A la figure 11, la mesure de résistance mécanique est effectuée pour une fibre 2 suivant l'invention dont la partie courbe 28 est constituée d'une spire et demie (appelée la spire 29 dans ce qui suit), faisant donc un tour et demi pour avoir les tronçons 23 et 27 parallèles en se faisant face. Pour réaliser cette mesure, la partie courbe 28 est immobilisée dans le plan de la figure 11 entre une deuxième mâchoire 202 et une première mâchoire 200, qui exerce une force F sur la partie courbe 28, tendant à faire se rapprocher les mâchoires 200 et 202. Ainsi, la spire 29 de la partie courbe 28 comporte un troisième point 281 de contact avec la première mâchoire 200 et un quatrième point 282 de contact avec la deuxième mâchoire 202, ce quatrième point 282 étant distinct du troisième point 281 et étant par exemple diamétralement opposé dans le plan de la spire par rapport au troisième point 281. Une force déterminée F de compression est appliquée sur le troisième point 281 de la partie courbe 28 par rapport au quatrième point 282. La force F appliquée sur la partie courbe 28 tend donc à faire se rapprocher les deux points distincts 281 et 282. La résistance mécanique à la compression de la partie courbe 28 est la valeur de la force F au-delà de laquelle la partie courbe 28 est brisée. Par conséquent, pour la valeur indiquée F de résistance mécanique, la partie courbe 28 de la fibre ne casse pas. La mesure de la force F est par exemple fournie par un capteur de force disposé sur un vérin faisant se rapprocher les mâchoires 200 et 202. Il a été utilisé pour cette mesure de résistance mécanique une machine de compression, à savoir un équipement Lloyd instrument LR50K avec une avance, c'est-à-dire une vitesse de rapprochement, de 10 mm / minute de la mâchoire 200 par rapport à la mâchoire 202.

On a ainsi mesuré à la figure 11, pour la fibre 2 dont la partie courbe 28 a une spire et demie 29 mise en forme selon le procédé de fabrication suivant l'invention, une résistance mécanique F = 1,5 N à la compression, cette fibre 2 suivant l'invention ayant par ailleurs les paramètres suivants :

- rayon de courbure de la spire et demie 29 de la partie courbe 28 : 1 mm ;
- épaisseur constante (diamètre de fibre) de la fibre sur toute sa longueur (tronçons 23, 25 et 27) : 240 $\mu$m ;
- composition de la fibre 2 : $Te_{20}As_{30}Se_{50}$ ;
- encombrement extérieur (hauteur) entre les points 281 et 282 : 2 mm.

Dans l'exemple comparatif de la figure 12, on a appliqué la même méthode de mesure qu'à là figure 11, mais sur une fibre 300 représentant l'art antérieur et non mise en forme selon le procédé suivant l'invention et courbée en U avec les paramètres suivants :

- rayon de courbure de la partie courbe 301 en U : 10 mm ;
- épaisseur constante (diamètre de fibre) de la fibre sur toute sa longueur (dans la partie courbe 301 et dans la fibre 300) : 240 $\mu$m;
- composition de la fibre 300 : $Te_{20}As_{30}Se_{50}$ ;
- encombrement extérieur (hauteur) entre les points 281 et 282 : 20mm;
- procédé de courbure de la fibre 300 selon l'art antérieur : on plie la fibre 300 à température ambiante (20°C environ) sans chauffage et sans enroulement.

On a ainsi mesuré une résistance mécanique à la compression, égale à F = 0.03 N à la figure 12.

Le capteur suivant l'invention possède donc intrinsèquement une résistance mécanique supérieure aux fibres courbées selon l'art antérieur pour une fibre de chalcogénure.

En effet, les fibres de chalcogénure sont connues dans l'art antérieur pour casser facilement, du fait de leur composition.

La tête de détection formée par la partie courbe 28 suivant l'invention est plus résistante et plus rigide, ce qui permet un meilleur contact avec le substrat étudié. Ainsi, la bonne résistance mécanique de la partie courbe 28 permet de manipuler facilement le capteur, notamment pour l'insérer dans toute gaine ou tube approprié ou plus généralement tout boîtier ou étui approprié pour son utilisation, et permet également de pouvoir appliquer et faire buter avec une certaine force la partie courbe 28 formant une tête de détection contre un substrat à consistance solide à étudier, afin d'être certain que la partie 25 de détection soit en contact avec ce substrat, comme par exemple un organe ou une partie d'un corps humain vivant ou d'un corps animal vivant.

D'une manière générale, la partie courbe 28 peut avoir un nombre quelconque n+1/2 spires 29, où n est un entier naturel supérieur ou égal à zéro, pour avoir les premier et troisième tronçons 23 et 27 se faisant face et par exemple sensiblement parallèles.

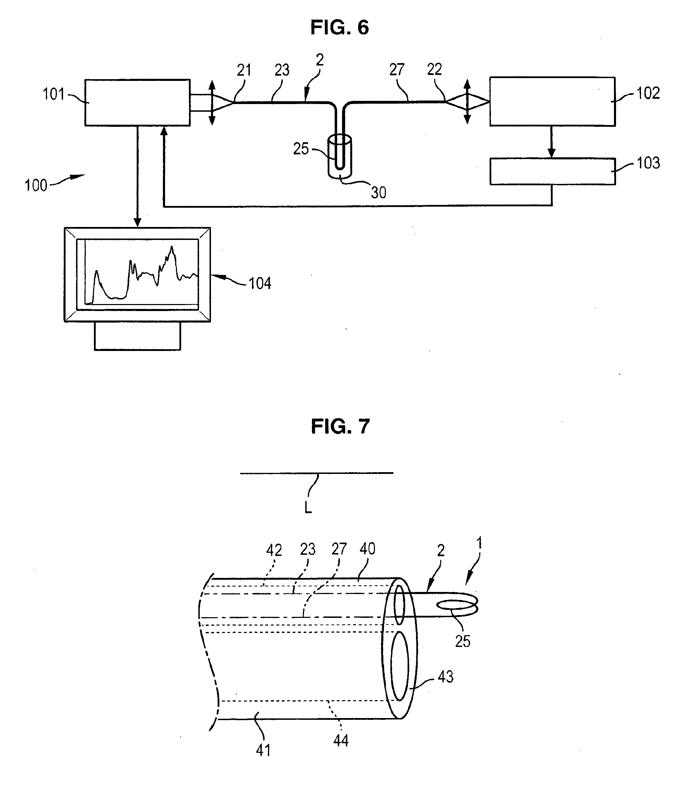

A la figure 6, un exemple de dispositif pour faire fonctionner le capteur suivant l'invention est représenté. Ce dispositif comporte un spectromètre infrarouge 101 relié à la première extrémité 21 de la fibre pour lui envoyer un signal infrarouge. Le deuxième tronçon 25 de détection de la fibre 2 est représenté en contact avec un échantillon 30 de laboratoire dans une éprouvette ou autre, c'est-à-dire à l'extérieur du corps humain ou animal. La deuxième extrémité 22 de la fibre 2 est reliée à un détecteur infrarouge 102 pour recevoir le signal infrarouge transmis de la première extrémité 21 à la deuxième extrémité 22 via les tronçons 23, 25, 27 de la fibre 2. Le détecteur 102 est relié à un amplificateur 103 du signal reçu par le détecteur 102. Le signal ayant été amplifié par l'amplificateur 103 est envoyé au spectromètre 101 comportant une unité 104 de traitement de signal permettant de comparer le signal infrarouge reçu sur l'extrémité 22 au signal infrarouge envoyé sur l'extrémité 21. Cette comparaison permet d'évaluer la perturbation apportée par l'échantillon 30 ou plus généralement le milieu extérieur sur le deuxième tronçon intermédiaire 25 de détection.

Ce procédé de détection permet de mettre en évidence par exemple sur l'échantillon 30 des anomalies métaboliques reflétant un état pathologique dans un sérum ou sur des biopsies hépatiques ou une contamination bactérienne d'un milieu organique.

Le spectromètre 101 émet dans l'infrarouge moyen. Le spectromètre comporte un algorithme d'analyse des spectres du signal envoyé et du signal reçu. Le signal envoyé peut également être dans l'infrarouge lointain. Le milieu extérieur avec lequel le tronçon intermédiaire 25 de détection est mis en contact peut être solide, liquide ou gazeux.

Par exemple, le premier tronçon 23 de fibre et le troisième tronçon 27 de fibre sont distants l'un de l'autre d'une largeur inférieure à 2 fois le rayon de courbure maximum de l'enroulement 28 transversalement à la longueur de la fibre et occupent un espace d'une largeur inférieure à 2 fois le rayon de courbure maximum de l'enroulement 28 transversalement à la longueur de la fibre.

Dans les modes de réalisation des figures 1, 3, 4, 5 et 7, les premiers et troisièmes tronçons 23, 37 se font face. Dans les modes de réalisation des figures 1 et 3, les premiers et troisièmes tronçons 23, 37 sont espacés l'un de l'autre d'une distance inférieure ou égale à deux fois le rayon de courbure maximum du deuxième tronçon 25 aux figures 1 et 3.

Les tronçons 23 et 27 sont par exemple parallèles. Le capteur 1 s'étend globalement entre un premier côté 10 où se trouvent les première et deuxième extrémités 21, 22 et un deuxième côté 11 où se trouve le deuxième tronçon 25 de détection, la fibre faisant un trajet aller du premier côté 10 au deuxième côté 11 par le premier tronçon 23 puis un trajet retour du deuxième côté 11 au premier côté 10 par le troisième tronçon 27. Bien entendu, la première ou deuxième extrémité 21, 22 peut dépasser du premier côté 10.

Dans le mode de réalisation représenté à la figure 7, le capteur 1 tel que décrit à la figure 1 est inséré dans un support 40. Ce support 40 est par exemple de forme allongé suivant la direction longitudinale L en étant par exemple en forme de gaine. Le support 40 comporte un corps 21 à l'intérieur duquel se trouve un conduit longitudinal 42 dans lequel sont insérés les premier et deuxième tronçons 23, 27 se trouvant face à face. Le conduit 42 a par exemple une largeur transversale supérieure ou égale à deux fois le rayon de courbure du deuxième tronçon 25 des figures 1 ou 3, ou supérieur ou égal à deux fois ce rayon de courbure auquel est ajoutée l'épaisseur transversale des deux tronçons 23, 27.

Le corps 41 et le conduit 42 s'étendent jusqu'à une extrémité 43 de détection. Le deuxième tronçon 25 de détection dépasse au moins en partie, et par exemple entièrement ainsi que représenté à la figure 7, en dehors de l'extrémité 43 de détection en dehors du conduit 42. Une partie des premier et deuxième tronçons 23, 27 peut également dépasser de l'extrémité 43 de détection. Le support 40 est par exemple en forme de tube. Le support 40 est par exemple un tube endoscopique d'exploration de l'intérieur du corps d'un être vivant. Ainsi, le support 40 endoscopique sert à amener le tronçon 25 de détection du capteur 1 contre un tissu vivant à l'intérieur d'un corps humain ou animal pour explorer celui-ci. En appuyant l'extrémité 43 de détection du tube contre le tissu vivant à l'intérieur du corps de l'être vivant, on appuie la partie 25 de détection du capteur 1 contre ce tissu vivant pour détecter les perturbations qu'il apporte à la propagation du signal infrarouge dans la fibre 2. Bien entendu, le support 40 peut comporter un ou plusieurs autres conduits 44 longitudinaux. Le capteur 1 de la figure 7 pourrait bien entendu être l'un des autres modes de réalisation décrits ci-dessus.

Le capteur 1 décrit ci-dessus peut être inséré dans le canal opératoire d'un dispositif médical, pour que la tête 28 de détection soit en contact avec un milieu ou un tissu biologique, pouvant être par exemple solide ou liquide, in vivo ou in vitro. Cela peut être par exemple dans un cathéter, dans un dispositif de diagnostic médical, à l'intérieur d'un être vivant ou contre un être vivant (par exemple la peau), dans un dispositif d'analyse médicale (par exemple analyse de sang in vitro).

Le capteur 1 peut ainsi servir à détecter dans le milieu extérieur la présence d'une ou plusieurs substances chimiques ayant un signature infrarouge dans le spectre de la fibre allant de 0.8 à 25 micromètres ou à mesurer la quantité de telles substances dans le milieu extérieur.

On a réalisé un test du capteur 1 selon la figure 1, ayant un enroulement 28 de 1 millimètre de rayon de courbure (diamètre de 2 millimètres) faisant une spire et demie, c'est-à-dire un tour à 360° et un demi-tour représentant une longueur équivalente L1 d'environ 5 millimètres. Avec ce capteur solénoïdal, on a mesuré le spectre de l'éthanol. La figure 10 représente en ordonnée l'absorbance en fonction de la longueur d'ondes en abscisse pour :

- la fibre 2 du capteur 1 sans éthanol, c'est-à-dire dans l'air, selon la première courbe C1,
- la fibre 2 du capteur 1 ayant son troisième tronçon 27 en contact avec l'éthanol en ayant son tronçon 25 dans l'air selon la deuxième courbe C2,
- la fibre 2 du capteur 1 ayant le deuxième tronçon 25 en contact avec l'éthanol sur son extrémité situé la plus du côté 11, c'est-à-dire sur la partie de l'enroulement 25 située la plus à droite à là figure 1.

On voit que la courbe C3 présente une très grande absorbance par rapport aux courbes C1 et C2. Le pic P à environ 9,5 micromètres correspond à une raie spectrale de l'éthanol, qui est donc mieux détectée par le capteur 1 suivant l'invention ayant le tronçon 25 de détection.

Grâce à la grande sensibilité de la tête 28 de détection suivant l'invention, on peut détecter des signatures moléculaires dans l'infrarouge dans le milieu extérieur avec lequel cette tête 28 est mise en contact, et en particulier détecter la présence de molécules en plus faibles quantités pour une même formule moléculaire et également un plus grand nombre de molécules de formules moléculaires différentes.

## Revendications

1. Procédé de fabrication d'un capteur (1) comprenant au moins une fibre (2) de chalcogénure permettant la propagation de la lumière infrarouge à au moins une longueur d'onde infrarouge comprise entre 0.8 à 25 micromètres et générant vers l'extérieur des ondes évanescentes pour détecter des signatures infrarouges d'un milieu extérieur, la fibre (2) de chalcogénure étant en une composition du type XY, où X est choisi parmi Ge, As ou Sb ou un mélange de deux ou de plus de deux de ces composants, et où Y est choisi parmi S, Se, Te ou un mélange de deux ou de plus de deux de ces composants, la fibre (2) de chalcogénure ayant une température Tg de transition vitreuse, la fibre comportant successivement sur sa longueur entre deux première et deuxième extrémités (21, 22) de la fibre (2) un premier tronçon (23) de fibre pour le guidage de l'onde infrarouge, au moins un deuxième tronçon (25) de fibre formant une partie intermédiaire ayant une fonction de détection et destiné à venir en contact avec le milieu extérieur pour détecter des signatures infrarouges perturbant la propagation des ondes évanescentes se propageant le long de la fibre (2), et un troisième tronçon (27) de fibre pour le guidage de l'onde infrarouge, **caractérisé en ce que** le procédé comprend les étapes consistant à :

   chauffer une âme (50) ayant une zone (53) de contact de dimensions transversales inférieures à 4.6 millimètres et appliquer la partie intermédiaire (25) de la fibre (2) contre ladite zone de contact de l'âme (50), la température de l'âme chauffée (50) étant telle que la partie intermédiaire (25) de la fibre (2) en contact avec la zone (53) de l'âme (50) ait une température T2 avec :

$$1,05 \,.\, Tg \leq T2 \leq 1,5 \,.\, Tg \,,$$

   et

   enrouler la partie intermédiaire (25) de la fibre (2) autour de la zone (53) de contact de l'âme (50) sur un angle d'enroulement d'au moins 180° de manière à former une partie courbe (28) dans ladite partie intermédiaire (25) de la fibre formant ledit deuxième tronçon (25) de la fibre avec un rayon de courbure localement inférieur à 2.3 millimètres.

2. Procédé de fabrication suivant la revendication 1, **caractérisé en ce que** l'âme (50) comporte des première et deuxième parties (51, 52), entre lesquelles se trouve ladite zone (53) de contact, l'âme étant chauffée par au moins l'une des première et deuxième parties (51, 52).

**3.** Procédé de fabrication suivant l'une quelconque des revendications 1 et 2, **caractérisé en ce que** l'âme (50) est cylindrique de section quelconque.

**4.** Procédé de fabrication suivant l'une quelconque des revendications 1 à 3, **caractérisé en ce que** la température T2 dépasse la température de transition vitreuse Tg de 10 % à 20 %.

**5.** Procédé de fabrication suivant l'une quelconque des revendications 1 à 4, **caractérisé en ce que** la partie intermédiaire (25) a un diamètre (D2) de fibre compris entre 50 et 450 micromètres.

**6.** Procédé suivant l'une quelconque des revendications 1 à 5, **caractérisé en ce que** la partie courbe (28) a une résistance mécanique à la compression, dans le sens visant à faire se rapprocher deux points distincts appartenant à la partie courbe (28), qui est supérieure ou égale à 1 N.

**7.** Procédé suivant l'une quelconque des revendications 1 à 6, **caractérisé en ce que** la partie courbe (28) comporte un enroulement comportant au moins une spire, la partie courbe (28) ayant une résistance mécanique à la compression, dans le sens visant à faire se rapprocher deux points distincts appartenant à la partie courbe (28), qui est supérieure ou égale à 1 N par spire.

**8.** Procédé suivant l'une des revendications 1 à 7, **caractérisé en ce que** ladite partie courbe a un rayon de courbure de fibre inférieur ou égal à 1 millimètre.

**9.** Procédé suivant l'une quelconque des revendications 1 à 8, **caractérisé en ce que** le premier tronçon (23) de fibre et le troisième tronçon (27) de fibre sont distants l'un de l'autre d'une largeur inférieure à 2,8 mm transversalement à la longueur de la fibre et occupent un espace d'une largeur inférieure à 2,8 mm transversalement à la longueur de la fibre.

**10.** Procédé suivant l'une quelconque des revendications 1 à 9, **caractérisé en ce que** la partie courbe (28) comporte un enroulement.

**11.** Procédé suivant l'une quelconque des revendications 1 à 10, **caractérisé en ce que** le deuxième tronçon (25) de détection présente au moins une dimension transversale (D1) de la fibre, inférieure à au moins une dimension transversale (D2) de la fibre dans les premier et troisième tronçons (23, 27).

**12.** Procédé suivant l'une quelconque des revendications 1 à 11, **caractérisé en ce que** la fibre a sur les premier, deuxième et troisième tronçons (23, 25, 27) une épaisseur de fibre comprise entre 50 et 450 micromètres et constante sur ces premier, deuxième et troisième tronçons (23, 25, 27).

**13.** Procédé suivant l'une quelconque des revendications 1 à 12, **caractérisé en ce que** les premier et troisième tronçons (23, 27) de fibre sont insérés dans une gaine de protection (40), le deuxième tronçon (25) dépassant au moins partiellement d'une extrémité (43) de la gaine et étant destiné à venir en contact avec le milieu étudié, qu'il soit solide et/ou liquide et/ou gazeux.

**14.** Procédé suivant l'une quelconque des revendications 1 à 13, **caractérisé en ce que** les premier et troisième tronçons (23, 27) de fibre sont insérés dans un canal opératoire d'un dispositif médical (40) de diagnostic, le deuxième tronçon (25) dépassant au moins partiellement d'une extrémité (43) du canal et étant destiné à venir en contact avec un tissu ou un liquide biologique in vivo et/ou ex vivo.

**15.** Procédé suivant l'une quelconque des revendications 1 à 14, **caractérisé en ce que** la proportion en poids de X est supérieure ou égale à 10% et inférieure ou égale à 70 %, tandis que la proportion en poids de Y est supérieure ou égale à 30 % et inférieure ou égale à 90 %.


**Patentansprüche**

**1.** Verfahren zur Herstellung eines Sensors (1), umfassend mindestens eine Chalcogenidfaser (2), die die Ausbreitung von Infrarotlicht auf mindestens einer Infrarotwellenlänge zwischen 0,8 bis 25 Mikrometer erlaubt und nach außen evaneszente Wellen erzeugt, um Infrarotsignaturen eines äußeren Mediums zu erfassen, wobei die Chalcogenidfaser (2) eine Zusammensetzung vom Typ XY hat, wobei X unter Ge, As oder Sb oder einem Gemisch von zwei oder mehr als zwei dieser Komponenten ausgewählt ist, und wobei Y unter S, Se, Te oder einem Gemisch aus zwei oder mehr als zwei dieser Komponenten ausgewählt ist, wobei die Chalcogenidfaser (2) eine Glasübergangstemperatur Tg hat, wobei die Faser nacheinander auf ihrer Länge zwischen zwei, einem ersten und einem zweiten, Enden (21, 22) der Faser (2) einen ersten Faserabschnitt (23) für die Führung der Infrarotwelle, mindestens einen zweiten Faserabschnitt (25), der einen Zwischenteil mit einer Erfassungsfunktion bildet und dazu bestimmt ist, mit dem äußeren Medium in Kontakt zu kommen, um Infrarotsignaturen zu erfassen, die die Ausbreitung der evaneszenten Wellen stören, die sich entlang der Faser (2) ausbreiten, und einen dritten Faserabschnitt (27) für die Führung der Infrarotwelle umfasst,

**dadurch gekennzeichnet, dass** das Verfahren die folgenden Schritte umfasst, bestehend aus:

- Erhitzen eines Kerns (50) mit einer Kontaktzone (53) mit Querabmessungen unter 4,6 Millimeter und Anlegen des Zwischenteils (25) der Faser (2) an die Kontaktzone des Kerns (50), wobei die Temperatur des erhitzten Kerns (50) derart ist, dass der Zwischenteil (25) der Faser (2), der mit der Zone (53) des Kerns (50) in Kontakt ist, eine Temperatur T2 bekommt, wobei:

$$1,05.\mathrm{Tg} \leq \mathrm{T2} \leq 1,5.\mathrm{Tg},$$

und
- Wickeln des Zwischenteils (25) der Faser (2) um die Kontaktzone (53) des Kerns (50) über einen Wickelwinkel von mindestens 180°, um einen gekrümmten Teil (28) in dem Zwischenteil (25) der Faser zu bilden, der den zweiten Abschnitt (25) der Faser mit einem Krümmungsradius bildet, der lokal kleiner als 2,3 Millimeter ist.

2. Herstellungsverfahren nach Anspruch 1, **dadurch gekennzeichnet, dass** der Kern (50) einen ersten und einen zweiten Teil (51, 52) umfasst, zwischen denen sich die Kontaktzone (53) befindet, wobei der Kern durch zumindest den ersten oder zweiten Teil (51, 52) erhitzt wird.

3. Herstellungsverfahren nach einem der Ansprüche 1 oder 2, **dadurch gekennzeichnet, dass** der Kern (50) zylindrisch mit einem beliebigen Querschnitt ist.

4. Herstellungsverfahren nach einem der Ansprüche 1 bis 3, **dadurch gekennzeichnet, dass** die Temperatur T2 die Glasübergangstemperatur Tg um 10% bis 20% überschreitet.

5. Herstellungsverfahren nach einem der Ansprüche 1 bis 4, **dadurch gekennzeichnet, dass** der Zwischenteil (25) einen Faserdurchmesser (D2) zwischen 50 und 450 Mikrometer hat.

6. Verfahren nach einem der Ansprüche 1 bis 5, **dadurch gekennzeichnet, dass** der gekrümmte Teil (28) in der Richtung, in der zwei verschiedene Punkte, die dem gekrümmten Teil (28) angehören, einander angenähert werden sollen, eine mechanische Druckfestigkeit aufweist, die größer oder gleich 1N ist.

7. Verfahren nach einem der Ansprüche 1 bis 6, **dadurch gekennzeichnet, dass** der gekrümmte Teil (28) eine Wicklung aufweist, umfassend mindestens eine Windung, wobei der gekrümmte Teil (28) in der Richtung, in der zwei verschiedene Punkte, die dem gekrümmten Teil (28) angehören, einander angenähert werden sollen, eine mechanische Druckfestigkeit aufweist, die größer oder gleich 1N ist.

8. Verfahren nach einem der Ansprüche 1 bis 7, **dadurch gekennzeichnet, dass** der gekrümmte Teil einen Faserkrümmungsradius kleiner oder gleich 1 Millimeter hat.

9. Verfahren nach einem der Ansprüche 1 bis 8, **dadurch gekennzeichnet, dass** der erste Faserabschnitt (23) und der dritte Faserabschnitt (27) voneinander um eine Breite kleiner 2,8 mm quer zur Länge der Faser entfernt sind und einen Raum mit einer Breite kleiner 2,8 mm quer zur Länge der Faser einnehmen.

10. Verfahren nach einem der Ansprüche 1 bis 9, **dadurch gekennzeichnet, dass** der gekrümmte Teil (28) eine Wicklung umfasst.

11. Verfahren nach einem der Ansprüche 1 bis 10, **dadurch gekennzeichnet, dass** der zweite Erfassungsabschnitt (25) zumindest eine Querabmessung (D1) der Faser aufweist, die kleiner als zumindest eine Querabmessung (D2) der Faser in dem ersten und dritten Abschnitt (23, 27) ist.

12. Verfahren nach einem der Ansprüche 1 bis 11, **dadurch gekennzeichnet, dass** die Faser über den ersten, zweiten und dritten Abschnitten (23, 25, 27) eine Faserdicke zwischen 50 und 450 Mikrometer hat, die über dem ersten, zweiten und dritten Abschnitt (23, 25, 27) jeweils konstant ist.

13. Verfahren nach einem der Ansprüche 1 bis 12, **dadurch gekennzeichnet, dass** der erste und der dritte Faserabschnitt (23, 27) in eine Schutzhülle (40) eingesetzt sind, wobei der zweite Abschnitt (25) zumindest teilweise über ein Ende (43) der Hülle hinausragt und dazu bestimmt ist, mit dem untersuchten Medium, sei es fest und/oder flüssig und/oder gasförmig, in Kontakt zu kommen.

14. Verfahren nach einem der Ansprüche 1 bis 13, **dadurch gekennzeichnet, dass** der erste und der dritte Faserabschnitt (23, 27) in einen Betriebskanal einer medizinischen Diagnoseeinrichtung (40) eingesetzt sind, wobei der zweite Abschnitt (25) zumindest teilweise über ein Ende (43) des Kanals hinausragt und dazu bestimmt ist, mit einem biologischen Gewebe oder einer biologischen Flüssigkeit in vivo und/oder ex vivo in Kontakt zu kommen.

15. Verfahren nach einem der Ansprüche 1 bis 14, **dadurch gekennzeichnet, dass** der Gewichtsanteil von X größer oder gleich 10% und kleiner oder gleich 70% ist, während der Gewichtsanteil von Y größer oder gleich 30 % und kleiner oder gleich 90 % ist.

**Claims**

1. A method for fabricating a sensor (1) comprising at least one chalcogenide fiber (2) allowing the propagation of infrared light at least at one infrared wavelength between 0.8 and 25 micrometers and outwardly generating evanescent waves to detect the infrared signatures of an outside medium, the chalcogenide fiber (2) having a composition of XY type where X is chosen from among Ge, As or Sb or a mixture of two or more than two of these components, and where Y is chosen from among S, Se, Te or a mixture of two or more than two of these components, the chalcogenide fiber (2) having a glass transition temperature Tg, the fiber successively comprising over its length between two first and second ends (21, 22) of the fiber (2) a first fiber section (23) for guiding the infrared wave, at least one second fiber section (25) forming an intermediate part having a detection function and intended to come into contact with the outside medium to detect infrared signatures perturbing the propagation of the evanescent waves propagating along the fiber (2), and a third fiber section (27) for guiding the infrared wave, **characterized in that** the method comprises the steps of:

heating a core (50) having a contact zone (53) of transverse dimensions smaller than 4.6 millimeters, and applying the intermediate part (25) of the fiber (2) against said contact zone of the core (50), the temperature of the heated core (50) being such that the intermediate part (25) of the fiber (2) in contact with the zone (53) of the core (50) has a temperature $T_2$ with:

$$1.05 \cdot T_g \leq T_2 \leq 1.5 \cdot T_g,$$

and
winding the intermediate part (25) of the fiber (2) around the contact zone (53) of the core (50) at a winding angle of at least 180° so as to form a bent part (28) in said intermediate part (25) of the fiber forming said second section (25) of the fiber with a radius of curvature locally smaller than 2.3 millimeters.

2. The fabrication method according to claim 1, **characterized in that** the core (50) comprises first and second portions (51, 52) between which there lies said contact zone (53), the core being heated by at least one of the first and second portions (51, 52).

3. The fabrication method according to any of claims 1 and 2, **characterized in that** the core (50) is cylindrical of any cross-section.

4. The fabrication method according to any of claims 1 to 3, **characterized in that** the temperature $T_2$ exceeds the

glass transition temperature Tg by 10% to 20%.

5.  The fabrication method according to any of claims 1 to 4, **characterized in that** the intermediate part (25) has a fiber diameter (D2) of between 50 and 450 micrometers.

6.  The method according to any of claims 1 to 5, **characterized in that** the bent part (28) has a mechanical compressive strength, in the direction in which it is sought to draw together two separate points belonging to the bent part (28), which is equal to or higher than 1 N.

7.  The method according to any of claims 1 to 6, **characterized in that** the bent part (28) comprises a winding comprising at least one turn, the bent part (28) having a mechanical compressive strength, in the direction in which it is sought to draw together two separate points belonging to the bent part (28), which is equal to or higher than 1 N per turn.

8.  The method according to any of claims 1 to 7, **characterized in that** said bent part has a fiber radius of curvature equal to or smaller than 1 millimeter.

9.  The method according to any of claims 1 to 8, **characterized in that** the first fiber section (23) and the third fiber section (27) are spaced apart by a width of less than 2.8 mm transverse to the length of the fiber and occupy a space of width smaller than 2.8 mm transverse to the length of the fiber.

10. The method according to any of claims 1 to 9, **characterized in that** the bent part (28) comprises a winding.

11. The method according to any of claims 1 to 10, **characterized in that** the second detection section (25) has at least one transverse dimension (D1) of the fiber that is smaller than at least one transverse dimension (D2) of the fiber in the first and third sections (23, 27).

12. The method according to any of claims 1 to 11, **characterized in that** on the first, second and third sections (23, 25, 27) the fiber has a thickness of between 50 and 450 micrometers that is constant over these first, second and third sections (23, 25, 27).

13. The method according to any of claims 1 to 12, **characterized in that** the first and third fiber sections (23, 27) are inserted in a protective sheath (40), the second section (25) projecting at least partly from one end (43) of the sheath and being intended to come into contact with the medium being examined, whether this be solid and/or liquid and/or gaseous.

14. The method according to any of claims 1 to 13, **characterized in that** the first and third fiber sections (23, 27) are inserted in an operating channel of a medical diagnosis device (40), the second section (25) projecting at least in part from one end (43) of the channel and being intended to come into contact with a tissue or biological fluid *in vivo* and/or *ex vivo*.

15. The method according to any of claims 1 to 14, **characterized in that** the proportion of X by weight is equal to or higher than 10% and equal to or lower than 70%, whilst the proportion of Y by weight is equal to or higher than 30% and equal to or lower than 90%.

## FIG. 1

## FIG. 2

## FIG. 3

## FIG. 4

## FIG. 5

## FIG. 6

## FIG. 7

**FIG. 8**

283

53  25
28

2

50

**FIG. 9**

2

23

51   25         50      52

53      28

27

**FIG. 10**

**Absorbance**

Longueur d'onde ($\mu$m)

200
28
29
202

F = 1,5 N
281
2
25
282

FIG. 11

200
301
202

F = 0,03 N
300
281
282

FIG. 12

## RÉFÉRENCES CITÉES DANS LA DESCRIPTION

**Documents brevets cités dans la description**

- US 5525800 A
- JP 7237932 B

**Littérature non-brevet citée dans la description**

- **J.KEIRSSE ; C. BOUSSARD-PLÉDEL ; O. LORÉAL ; O. SIRE ; B. BUREAU ; P. LEROYER ; B. TURLIN ; J. LUCAS.** IR optical fiber sensor for biomédical applications. *Vibrational Spectroscopy,* 2003, vol. 32, 23-32
- **PIERRE LUCAS ; MARK R. RILEY ; CATHERINE BOUSSARD-PLÉDEL ; BRUNO BUREAU.** Advances in chalcogenide fiber evanescent wave biochemical sensing. *Analytical Biochemistry,* 2006, 1-10
- **LITTLEJOHN D et al.** BENT SILICA FIBER EVANESCENT ABSORPTION SENSORS FOR NEAR-INFRARED SPECTROSCOPY. *BENT SILICA FIBER EVANESCENT ABSORPTION SENSORS FOR NEAR-INFRARED SPECTROSCOPY,* 01 Juillet 1999, vol. 53 (7), 845-849
- Recent developments in chemical sensing using infrared glass fibers. **HOCDE S et al.** JOURNAL OF NON-CRYSTALLINE SOLIDS. NORTH-HOLLAND PHYSICS PUBLISHING, 01 Septembre 2000, vol. 274, 17-22